# EUROPEAN PATENT SPECIFICATION

(11) **EP 3 345 608 B1**
(45) Date of publication and mention of the grant of the patent: **27.11.2024**
(21) Application number: 16842331.7
(22) Date of filing: 01.09.2016
(51) Int. Cl.: A61K 31/7004, A61P 3/06

(54) **METHOD FOR INHIBITING ABSORPTION OF AND PROMOTING EXCRETION OF LIPIDS USING D-PSICOSE**
VERFAHREN ZUR INHIBIERUNG DER ABSORPTION DER UND FÖRDERUNG DER AUSSCHEIDUNG VON LIPIDEN MITTELS D-PSICOSE
MÉTHODE D'INHIBITION DE L'ABSORPTION ET DE PROMOTION DE L'EXCRÉTION DE LIPIDES À L'AIDE DE D-PSICOSE

(30) Priority: 01.09.2015 KR 20150123437
(43) Date of publication of application: 11.07.2018
(73) Proprietor: CJ Cheiljedang Corporation, Seoul 04560 (KR)
(72) Inventor: CHOI, Myung-Sook, Daegu 42170 (KR); KWON, Eun-young, Gyeonggi-do 10323 (KR); HAN, Youngji, Chilgok-gun Gyeongsangbuk-do 39891 (KR)
(74) Representative: Cabinet Beau de Loménie
(86) International application number: PCT/KR2016/009812
(87) International publication number: WO 2017/039365

(56) References cited:
- EP-A1- 1 757 297
- EP-A1- 2 156 751
- EP-A1- 2 992 887
- WO-A1-2007/010976
- WO-A1-2014/175119
- JP-A- 2010 018 528
- KOUICHI ITOH ET AL: "Beneficial Effects of Supplementation of the Rare Sugar "D-allulose" Against Hepatic Steatosis and Severe Obesity in Lep ob / Lep ob Mice : D-allulose improves fat liver in obesity...", JOURNAL OF FOOD SCIENCE, vol. 80, no. 7, July 2015 (2015-07-01), US, pages H1619 - H1626, XP055571021, ISSN: 0022-1147, DOI: 10.1111/1750-3841.12908
- BAEK ET AL: "D-Psicose, a Sweet Monosaccharide, Ameliorate Hyperglycemia, and Dyslipidemia in C57BL/6Jdb/dbMice", JOURNAL OF FOOD SCIENCE, WILEY-BLACKWELL PUBLISHING, INC, US, vol. 75, no. 2, March 2010 (2010-03-01), pages H49 - H53, XP009511230, ISSN: 0022-1147, Retrieved from the Internet <URL:https://onlinelibrary.wiley.com/doi/10.1111/j.1750-3841.2009.01434.x/full> [retrieved on 20100301], DOI: 10.1111/J.1750-3841.2009.01434.X
- AKRAM HOSSAIN ET AL: "Rare sugar d-allulose: Potential role and therapeutic monitoring in maintaining obesity and type 2 diabetes mellitus", PHARMACOLOGY AND THERAPEUTICS., vol. 155, 20 August 2015 (2015-08-20), GB, pages 49 - 59, XP055571013, ISSN: 0163-7258, DOI: 10.1016/j.pharmthera.2015.08.004
- TATSUHIRO MATSUO ET AL: "Dietary D-psicose, a C-3 epimer of D-fructose, suppresses the activity of hepatic lipogenic enzymes in rats", ASIA PACIFIC JOURNAL OF CLINICAL NUTRITION, vol. 10, no. 3, 27 September 2001 (2001-09-27), GB, pages 233 - 237, XP055571040, ISSN: 0964-7058, DOI: 10.1046/j.1440-6047.2001.00246.x
- YOUNG-MEE CHUNG ET AL: "Dietary D-Psicose Reduced Visceral Fat Mass in High-Fat Diet-Induced Obese Rats", JOURNAL OF FOOD SCIENCE, vol. 77, no. 2, 1 February 2012 (2012-02-01), US, pages H53 - H58, XP055570593, ISSN: 0022-1147, DOI: 10.1111/j.1750-3841.2011.02571.x
- OCHIAI, M. ET AL.: "Inhibition by Dietary d-psicose of Body Fat Accumulation in Adult Rats Fed a High-sucrose Diet", BIOSCIENCE, BIOTECHNOLOGY, AND BIOCHEMISTRY, vol. 77, no. 5, 2013, pages 1123 - 1126, XP055219362
- HOSSAIN, A. ET AL.: "Rare Sugar d-psicose Prevents Progression and Development of Diabetes in T2DM Model Otsuka Long-evans Tokushima Fatty Rats", DRUG DESIGN, DEVELOPMENT AND THERAPY, vol. 9, January 2015 (2015-01-01), pages 525 - 535, XP055267918
- NAGATA, Y. ET AL.: "d-Psicose, an Epimer of d-fructose, Favorably Alters Lipid Metabolism in Sprague-Dawley Rats", JOURNAL OF AGRICULTURAL AND FOOD CHEMISTRY, vol. 63, March 2015 (2015-03-01), pages 3168 - 3176, XP055517512
- OCHIAI, M. ET AL.: "d-Psicose Increases Energy Expenditure and Decreases Body Fat Accumulation in Rats Fed a High-sucrose Diet", INTERNATIONAL JOURNAL OF FOOD SCIENCES AND NUTRITION, vol. 65, no. 2, 2014, pages 245 - 250, XP055289129

## Description

### [Technical Field]

The present invention relates to a method for inhibiting lipid absorption and/or promoting lipid excretion using D-psicose.

### [Background Art]

D-psicose, the C-3 epimer of D-fructose, is a natural sugar present in a trace amount in commercial mixtures of D-glucose and D-fructose obtained from hydrolysis of sucrose or isomerization of D-glucose. D-psicose is a monosaccharide with a sweetness of 70% relative to sugar. D-psicose was reported to be a sweetener that contains few or no calories because it is not metabolized in a body and that has little effect on body weight gain because it functions to inhibit the formation of body fat. According to a recently published report, D-psicose has non-cariogenic and anti-cariogenic effects. Under these circumstances, D-psicose is currently under active development as a sweetener that has the potential to replace sugar while assisting in dental health. Thus, D-psicose has received attention as a sweetener for preventing weight gain in the food industry due to characteristics and functionalities thereof.

D-psicose is generally recognized as safe (GRAS) by the United States Department of Agriculture (USDA). Some studies reported that D-psicose affects lipid metabolism (Yasuo nagata et al., J. Agric, Food Chem. 2015, 63, 3168-3176), but, to the best of our knowledge, no report on the use of D-psicose for reducing lipid absorption and promoting lipid excretion has been published to date.

KOUICHI ITOH ET AL: "Beneficial Effects of Supplementation of the Rare Sugar "D- allulose" Against Hepatic Steatosis and Severe Obesity in Lep ob / Lep ob Mice", JOURNAL OF FOOD SCIENCE, vol. 80, no. 7, July 2015 mentions that D-psicose is useful against hyperlipidemia and it shows that it improves hepatic steatosis (fatty liver).

EP 1 757 297 A1 discloses the use of D-psicose as antiatherogenic for inhibiting the onset of arteriosclerosis.

BAEK ET AL: "D-Psicose, a Sweet Monosaccharide, Ameliorate Hyperglycemia, and Dyslipidemia in C57BL/6Jdb/dbMice", JOURNAL OF FOOD SCIENCE, vol. 75, no. 2, March 2010 (2010-03), shows D-psicose ameliorates the ratio LDL/HDL in plasma in a genetic diabetes model in mice.

AKRAM HOSSAIN ET AL: "Rare sugar d-allulose: Potential role and therapeutic monitoring in maintaining obesity and type 2 diabetes mellitus", PHARMACOLOGY AND THERAPEUTICS., vol. 155, 20 August 2015 (2015-08-20), teaches that D-psicose has a strong anti-hyperlipidemic effect and is thus useful in the prevention of atherosclerosis.

TATSUHIRO MATSUO ET AL: "Dietary D-psicose, a C-3 epimer of D-fructose, suppresses the activity of hepatic lipogenic enzymes in rats", ASIA PACIFIC JOURNAL OF CLINICAL NUTRITION, vol. 10, no. 3, 27 September 2001 (2001-09-27), teaches that D-psicose suppresses FAS activity in the liver.

WO2007/010976 A1 discloses the use of a composition comprising D-psicose for suppressing an excessive increase in body weight and/or reducing overweight.

EP2156751 A1 shows that the administration of D-psicose results in a significant decrease in body weight and body fat weight.

WO2014/175119A1 teaches that the energy expenditure and the lipid combustion significantly increased when D-psicose was given immediately before eating the common lipid-containing food. This document discloses the use of a composition comprising D-psicose to reduce excessive body weight and visceral fat accumulation.

EP2992887A1 shows that the use of D-psicose results in reduced weight gain, food efficiency ratio, and fat accumulation in high-fat diet-induced obese rats than the control groups.

Young-Mee Chung et al.: "Dietary Dpsicose Reduced Viceral Fat Mass in High-Fat Diet-Induced Obese Rats", Journal of Food Science, vol. 77, no. 2, 1 February 2012, shows that the use of D-psicose results in reduced weight gain, food efficiency ratio, and fat accumulation in high-fat diet-induced obese rats than the control groups.

NAGATA, Y. et AL.: "D-psicose, an Epimer of D-Fructose, Favorably Alters Lipid Metabolism in Sprague-Dawley Rats", JOURNAL OF AGRICULTURAL AND FOOD CHEMISTRY, vol. 63, no. 12, 1 January 2015, discloses that there are no significant differences in the levels of serum triglyceride, non-esterified fatty acid, and serum glucose, and further discloses that the effect s of D-psicose vary depending on the tissue. Additionally, this document discloses that the levels of liver cholesterol and triglyceride are not significantly different throughout consistent observations. This document also discloses that the level of gene expression of proteins involved in lipid metabolism is not significantly different in certain tissues.

The present inventors have found the fact that D-psicose has functions of inhibiting lipid absorption in the small intestine and considerably increasing lipid levels in feces, reduces body weight, body fat mass, and plasma levels of lipids (including free fatty acids, triglycerides, total cholesterol, non-high-density lipoprotein cholesterol, and apolipoprotein B) such that body weight, body fat mass, and plasma lipid profiles are normalized, and is effective in reducing the activity of fatty acid synthase (FAS). The present invention has been accomplished based on this finding.

### [Disclosure]

### [Technical Problem]

It is an aspect of the present invention to provide D-psicose for use in inhibiting the absorption of lipids ingested by a subject and promoting the excretion of the ingested lipids in a subject, thereby preventing hyperlipidemia, arteriosclerosis or fatty liver in the subject, wherein the D-psicose is administered in an amount of 10 to 50 parts by weight, relative to 100 parts by weight of the lipids ingested by the subject.

It is another aspect of the present invention to provide a composition comprising D-psicose for use in inhibiting the absorption of lipids ingested by a subject and promoting the excretion of the ingested lipids in a subject, thereby preventing hyperlipidemia, arteriosclerosis or fatty liver in the subject, wherein the D-psicose is administered in an amount of 10 to 50 parts by weight, relative to 100 parts by weight of the lipids ingested by the subject.

The present invention will be now described in more detail. Disclosures that are not included herein will be readily recognized and appreciated by those skilled in the art, and thus a description thereof is omitted.

### [Technical Solution]

One aspect of the present invention provides D-psicose for use in inhibiting the absorption of lipids ingested by a subject and promoting the excretion of the ingested lipids in a subject, thereby preventing hyperlipidemia, arteriosclerosis or fatty liver in the subject, wherein the D-psicose is administered in an amount of 10 to 50 parts by weight, relative to 100 parts by weight of the lipids ingested by the subject.

Another aspect of the present invention provides a composition comprising D-psicose for use in inhibiting the absorption of lipids ingested by a subject and promoting the excretion of the ingested lipids in a subject, thereby preventing hyperlipidemia, arteriosclerosis or fatty liver in the subject, wherein the D-psicose is administered in an amount of 10 to 50 parts by weight, relative to 100 parts by weight of the lipids ingested by the subject.

The subject includes mammals including humans and non-human mammals. Examples of the non-human mammals include, but are not limited to, mice, rats, dogs, cats, horses, cows, sheep, goats, pigs, and rabbits.

The lipids include animal lipids and vegetable lipids but are not limited thereto. Specifically, the lipids may be animal lipids, vegetable lipids or combinations thereof. More specifically, the lipids may be ones that are present in food or feed.

The administration may be oral administration or parenteral administration (e.g., intravenous administration, subcutaneous administration, intraperitoneal administration or topical application). Specifically, the administration may be oral administration.

D-psicose is administered in an amount of 10 to 50 parts by weight, relative to 100 parts by weight of the lipids ingested by the subject. More specifically, D-psicose may be administered in an amount of 10-40, 10-30, 10-25, 15-50, 15-40, 15-30, 15-25, 20-50, 20-40, 20-30, 20-25 or 25 parts by weight, relative to 100 parts by weight of the lipids ingested by the subject.

In one embodiment of the present invention, the absorption may be absorption in the small intestine. Specifically, when administered to high-fat diet-fed obese mice, D-psicose reduces mRNA expression of genes (CD36, FATP4, and ApoB48) involved in lipid absorption in the small intestine. Specifically, D-psicose reduces mRNA expression of genes (ABCG5 and ABCG8) involved in lipid excretion.

A further aspect not according to the present invention provides use of a composition comprising D-psicose for inhibiting the absorption of food lipids and/or promoting the excretion of food lipids.

To the best of our knowledge, this is the first report on the mechanism by which D-psicose, which is widely used as a sweetener, inhibits the absorption of food lipids and promotes the excretion of food lipids. Details of the mechanism are the same as those described above.

Yet another aspect not according to the present invention provides a method for inhibiting the activity of fatty acid synthase (FAS) in a subject comprising administering a fatty acid synthase inhibitor comprising D-psicose or D-psicose to the subject.

In one embodiment of the present invention, D-psicose reduces fatty acid β-oxidation activity in the liver. Alternatively, D-psicose may induce fatty acid β-oxidation activity in adipose tissue.

Yet another aspect not according to the present invention provides a method for preventing, ameliorating or treating hyperlipidemia, arteriosclerosis or fatty liver comprising administering a pharmaceutically effective amount of D-psicose to a subject in need of such prevention, amelioration or treatment.

Yet another aspect not according to the present invention provides a composition for preventing, ameliorating or treating hyperlipidemia, arteriosclerosis or fatty liver comprising D-psicose.

As used herein, the term "hyperlipidemia" refers to a disease caused by abnormally high blood fat levels as a result of insufficient metabolism of fats such as triglycerides and cholesterol. More specifically, hyperlipidemia is characterized by increased levels of lipids (including triglycerides, LDL cholesterol, phospholipids, and free fatty acids) in the blood, and hyperlipidemia is including hypercholesterinemia or hypertriglyceridemia, which occurs frequently from increased levels of lipids.

As used herein, the term "arteriosclerosis" refers to a disease where cholesterol is deposited on the inner walls of the arteries or vascular endothelial cells proliferate to narrow or occlude the arteries, causing poor blood circulation to the peripheries.

As used herein, the term "fatty liver" refers to a condition where fat accumulates excessively in hepatic cells due to the disorder of fat metabolism in the liver. Fatty liver is a cause of various diseases such as angina, myocardial infarction, stroke, arteriosclerosis, fatty liver and pancreatitis.

As used herein, the term "prevention" or "preventing" means all actions that inhibit or delay the development of target diseases. Specifically, this term means administering D-psicose to inhibit or delay the development of hyperlipidemia, arteriosclerosis, and fatty liver symptoms (for example, elevated plasma free fatty acid, triglyceride, total cholesterol, non-HDL cholesterol, and Apo B levels, high arteriosclerosis index (AI), increased fatty acid, triglyceride, and cholesterol levels in hepatic tissue, and increased size of adipocytes).

As used herein, the term "amelioration" or "ameliorating" means all actions that alleviate or relieve symptoms and side effects of diseases. As used herein, the term "treatment" or "treating" refers to all actions that alleviate or beneficially change symptoms and side effects of diseases. Specifically, these terms mean administering D-psicose to alleviate, palliate or relieve hyperlipidemia, arteriosclerosis or fatty liver symptoms, resulting in reduced plasma free fatty acid, triglyceride, total cholesterol, non-HDL cholesterol, or Apo B level, low arteriosclerosis index (AI), reduced fatty acid, triglyceride or cholesterol level or reduced size of adipocytes in hepatic tissue.

As demonstrated in Examples section that follows, it was found that D-psicose significantly reduces free fatty acids, triglyceride, total cholesterol, non-HDL cholesterol, Apo B, leptin, resistin levels and leptin/adiponectin ratio in the plasma of high-fat diet-induced obese mice such that the levels and ratio are maintained similarly to those in the normal diet group, increases the levels of plasma HDL-cholesterol and Apo A-1 to higher values than those in the normal diet group, and lowers arteriosclerosis index (AI), thus being effective in preventing, ameliorating or treating hyperlipidemia or arteriosclerosis. It was also found that D-psicose reduces the activity of fatty acid synthase (FAS), the levels of fatty acids, triglycerides, cholesterol, and the size of adipocytes in the liver tissues of high-fat diet-induced obese mice to inhibit the development of fatty liver by high-fat diet. Furthermore, D-psicose was confirmed to reduce mRNA expression of genes involved in fatty acid synthesis in the livers of high-fat diet-induced obese mice, thus being effective in preventing or treating fatty liver.

Based on these findings, it can be concluded that D-psicose has functions of inhibiting lipid absorption in the small intestine and considerably increasing lipid levels in feces and is effective in reducing plasma lipid level to normalize plasma lipid profiles. Therefore, D-psicose can find application in pharmaceutical drugs and foods (specifically, health functional foods) for preventing, ameliorating or treating hyperlipidemia, arteriosclerosis or fatty liver.

The composition for use according to the present invention can be administered orally or parenterally (e.g., intravenously, subcutaneously, intraperitoneally or topically) depending on the intended use. Specifically, the composition for use according to the present invention can be administered orally.

The composition for use according to the present invention may be used as a pharmaceutical composition. In this case, the composition for use according to the present invention may further comprise at least one pharmaceutically acceptable carrier suitable for administration. The pharmaceutically acceptable carrier may be used in admixture with one or more components selected from saline solution, sterilized water, Ringer's solution, buffered saline, dextrose solution, maltodextrin solution, glycerol, and ethanol. One or more general additives, such as antioxidants, buffer solutions, and bacteriostatic agents may be added, if needed. The composition for use according to the present invention may be prepared into injectable formulations (such as aqueous solutions, suspensions or emulsions), pills, capsules, granules, or tablets. In this case, the composition for use according to the present invention may further comprise one or more additives selected from diluents, dispersants, surfactants, binders, and lubricants. The composition for use according to the present invention may be prepared into various formulations depending on the type of disease or the kind of components according to any suitable method known in the art or any of the conventional procedures disclosed in Remington's Pharmaceutical Science (the newest edition), Mack Publishing Company, Easton PA.

The dose of the pharmaceutical composition for use according to the present invention may be determined taking into consideration various factors, including body weight, age, sex, health condition, diet, time and mode of administration, and rate of excretion, and severity of disease. A daily dose of D-psicose may be range from about 0.0001 to about 600 mg/kg, preferably about 0.001 to about 500 mg/kg, and may be administered in single or divided doses per day.

The pharmaceutical composition for use according to the present invention may be used alone or in combination with surgical operation, hormone therapy, drug treatment, and biological regulators.

The composition for use according to the present invention may be used as a food or health food composition. In this case, D-psicose may be added as it is or in combination with other foods or food ingredients and may be suitably used according to any general method known in the art. The amount of the active ingredient can be determined according to the purpose of use (prevention, health or therapeutic regimen). The food composition may be used without limitation in any food or health food that includes lipids. Examples of suitable foods include meats, sausages, breads, cakes, chocolates, candies, snacks, crackers, cookies, pizza, flour products (e.g., instant noodles), gums, dairy products (including ice creams), soups, ketchups, sauces, gravies, dressings, beverages, teas, drinks, alcoholic drinks, and vitamin complexes.

The food or health food composition for use according to the present invention may further comprise various flavors or natural carbohydrates, like general beverages. The natural carbohydrates include monosaccharides such as glucose and fructose, disaccharides such as maltose and sucrose, polysaccharides such as dextrin and cyclodextrin, and sugar alcohols such as xylitol, sorbitol, and erythritol. The food or health food composition for use according to the present invention may further comprise natural or synthetic sweetening agents. The natural sweetening agents include thaumatin and stevia extracts. The synthetic sweetening agents include saccharin and aspartame. The natural carbohydrate is typically used in an amount of about 0.01 to about 0.20 g, specifically 0.04 to 0.10 g, per 100 ml of the food or health food composition.

The food or health food composition for use according to the present invention may further comprise a variety of nutrients, vitamins, electrolytes, flavors, coloring agents, pectic acid and its salts, alginic acid and its salts, organic acids, protective colloidal thickeners, pH adjusting agents, stabilizers, preservatives, glycerin, alcohols, and carbonating agent for carbonated beverages. The food or health food composition for use according to the present invention may further comprise fruit flesh for the production of natural fruit juices, fruit juice beverages and/or vegetable beverages. These ingredients may be used independently or in combination. The total amount of the ingredients added may be in the range of 0.01 to 0.20 parts by weight, relative to 100 parts by weight of the food or health food composition.

The use of reducing the absorption of food lipids, the inhibitor of fatty acid synthase activity, the method for inhibiting the activity of fatty acid synthase, the method for preventing, ameliorating or treating hyperlipidemia, arteriosclerosis or fatty liver, and the composition according to the present description not according to the present invention share D-psicose, lipid, administration, and subject in common with the method for inhibiting lipid absorption and/or promoting lipid absorption, and a description thereof is thus omitted to avoid excessive complexity of the specification.

### [Advantageous Effects]

The present inventors have attempted to clarify the physiological activity of D-psicose by assigning isocaloric diets to each diet group to exclude the effect of D-psicose on calorie reduction, and as a result, found that D-psicose has functions of inhibiting lipid absorption in the small intestine and considerably increasing lipid levels in feces to inhibit fat production and reduces body weight, body fat mass, and plasma lipid levels such that body weight, body fat mass, and plasma lipid profiles are normalized in a short time. Due to these advantages, it is expected that D-psicose will be used to prevent and/or treat lipid-related metabolic diseases.

### [Description of Drawings]

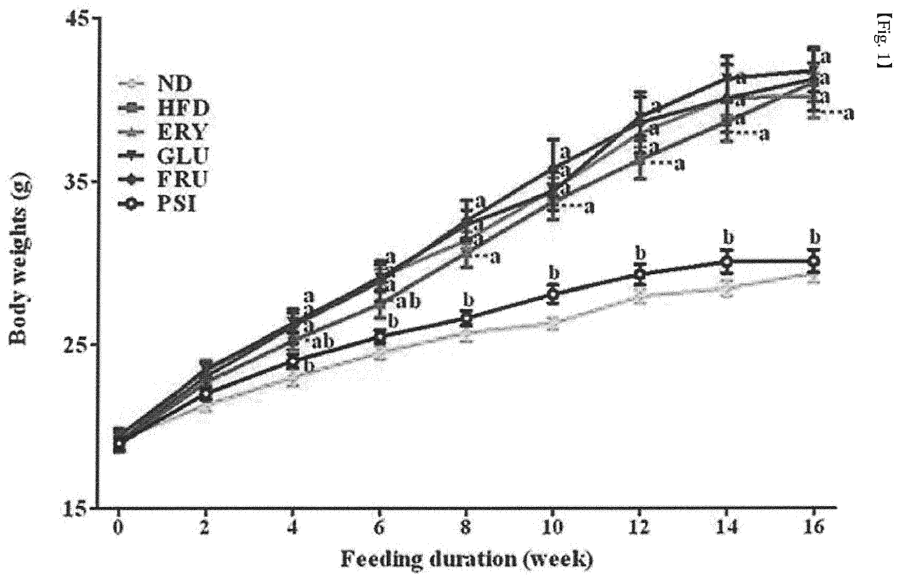

Fig. 1 shows changes in the body weight of C57BL/6J mice fed both D-psicose and high-fat diet for 16 weeks [normal diet group (ND), high-fat diet group (HFD), PSI group (HFD + 5% D-psicose, w/w), ERY group (HFD + 5% erythritol, w/w), GLU group (HFD + 5% D-glucose, w/w), and FRU group (HFD + 5% D-fructose, w/w)].

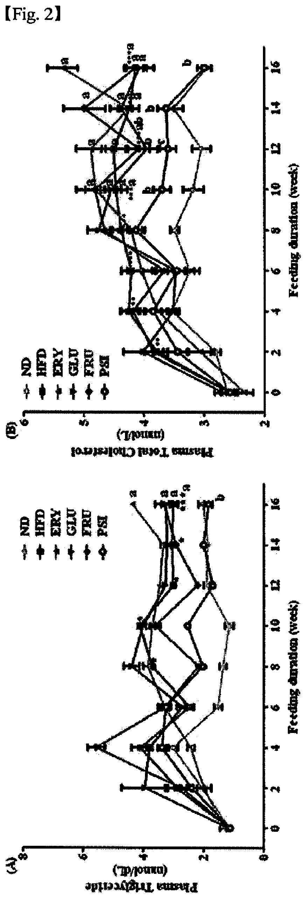

Fig. 2 shows changes in plasma triglyceride and total cholesterol levels in C57BL/6J mice fed both D-psicose and high-fat diet for 16 weeks [normal diet group (ND), high-fat diet group (HFD), PSI group (HFD + 5% D-psicose, w/w), ERY group (HFD + 5% erythritol, w/w), GLU group (HFD + 5% D-glucose, w/w), and FRU group (HFD + 5% D-fructose, w/w)].

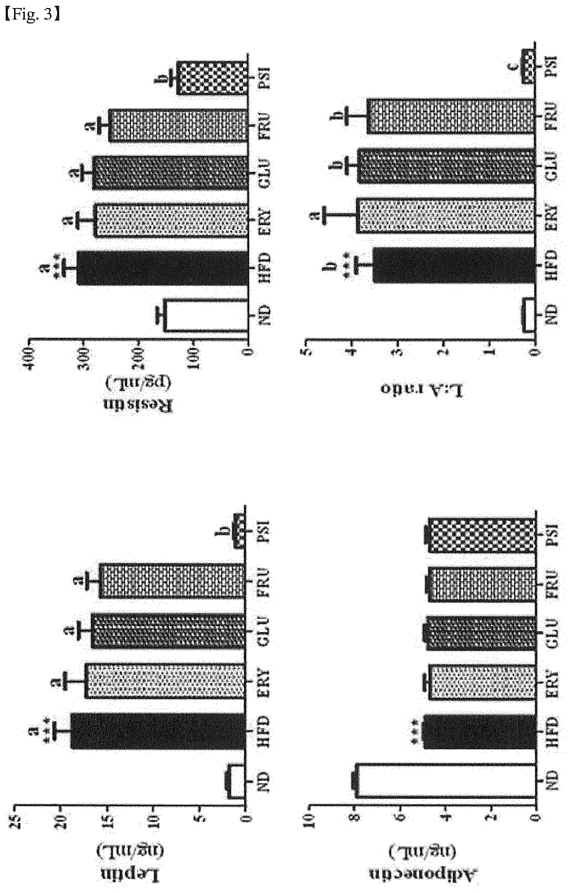

Fig. 3 shows the influences of D-psicose on plasma leptin, resistin, and adiponectin levels, and leptin:adiponectin ratio (L:A ratio) in high-fat diet-induced obese mice [normal diet group (ND), high-fat diet group (HFD), PSI group (HFD + 5% D-psicose, w/w), ERY group (HFD + 5% erythritol, w/w), GLU group (HFD + 5% D-glucose, w/w), and FRU group (HFD + 5% D-fructose, w/w)].

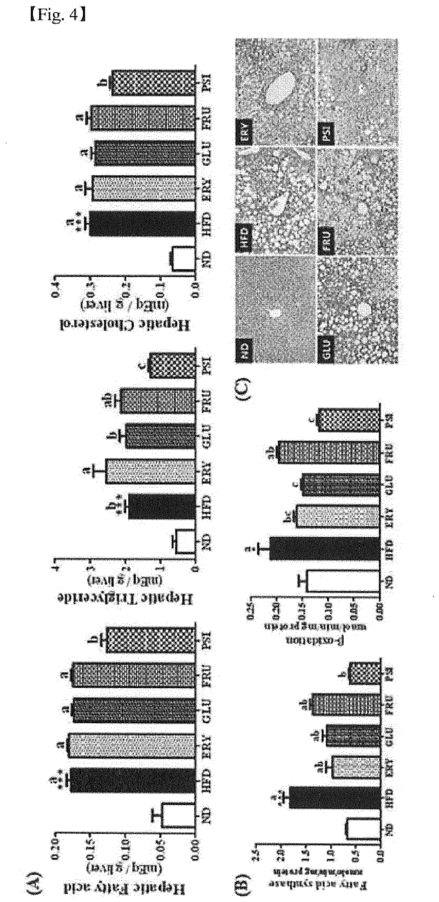

Fig. 4 shows the influences of D-psicose on (A) hepatic lipid profiles, (B) hepatic lipid regulating enzyme activities, and (C) hepatic tissue morphologies of high-fat diet-induced obese mice [normal diet group (ND), high-fat diet group (HFD), PSI group (HFD + 5% D-psicose, w/w), ERY group (HFD + 5% erythritol, w/w), GLU group (HFD + 5% D-glucose, w/w), and FRU group (HFD + 5% D-fructose, w/w)].

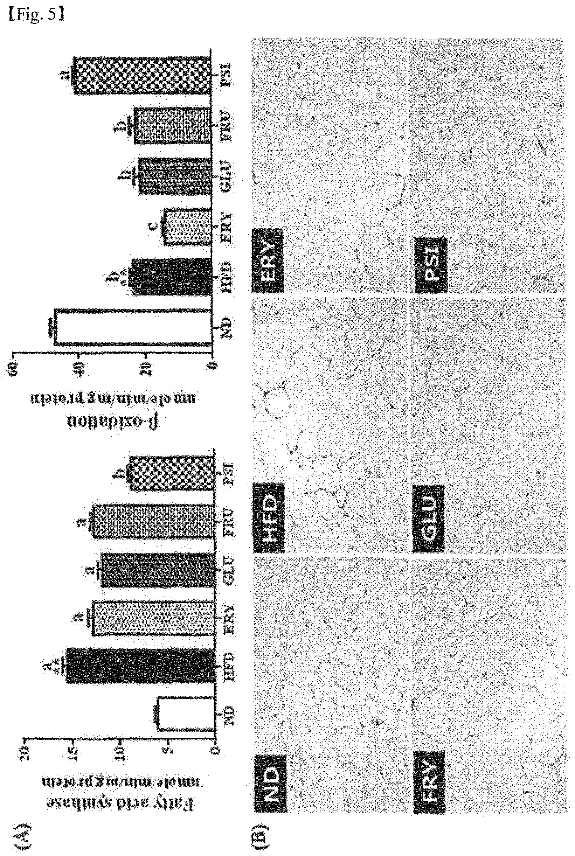

Fig. 5 shows the influences of D-psicose on (A) hepatic lipid regulating enzyme activities and (B) hepatic tissue morphologies of high-fat diet-induced obese mice [normal diet group (ND), high-fat diet group (HFD), PSI group (HFD + 5% D-psicose, w/w), ERY group (HFD + 5% erythritol, w/w), GLU group (HFD + 5% D-glucose, w/w), and FRU group (HFD + 5% D-fructose, w/w)].

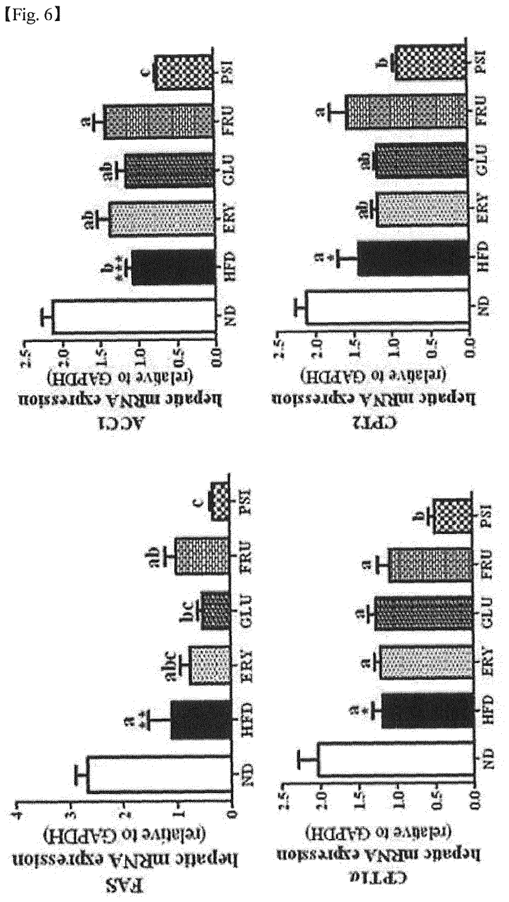

Fig. 6 shows the influences of D-psicose on mRNA expression of genes (FAS, ACC1, CPT1α, and CPT2) involved in fatty acid synthesis and oxidation in the livers of high-fat diet-induced obese mice [normal diet group (ND), high-fat diet group (HFD), PSI group (HFD + 5% D-psicose, w/w), ERY group (HFD + 5% erythritol, w/w), GLU group (HFD + 5% D-glucose, w/w), and FRU group (HFD + 5% D-fructose, w/w)].

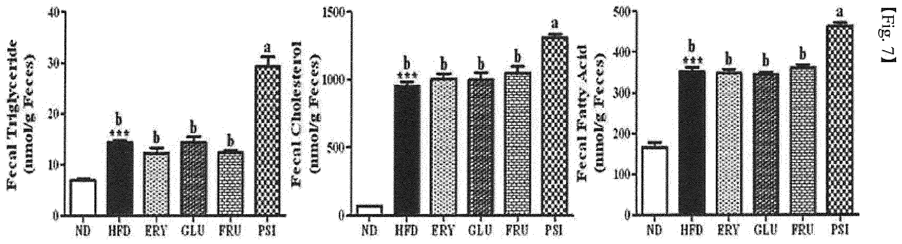

Fig. 7 shows the influences of D-psicose on lipid levels in feces from high-fat diet-induced obese mice [normal diet group (ND), high-fat diet group (HFD), PSI group (HFD + 5% D-psicose, w/w), ERY group (HFD + 5% erythritol, w/w), GLU group (HFD + 5% D-glucose, w/w), and FRU group (HFD + 5% D-fructose, w/w)].

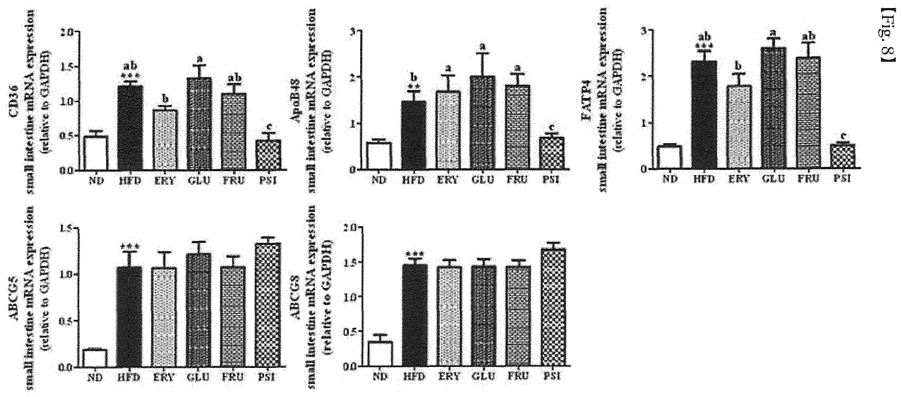

Fig. 8 shows the influences of D-psicose on mRNA expression of genes (CD36, FATP4, ApoB48, ABCG5 and ABCG8) involved in lipid absorption and excretion in the small intestines of high-fat diet-induced obese mice [normal diet group (ND), high-fat diet group (HFD), PSI group (HFD + 5% D-psicose, w/w), ERY group (HFD + 5% erythritol, w/w), GLU group (HFD + 5% D-glucose, w/w), and FRU group (HFD + 5% D-fructose, w/w)].

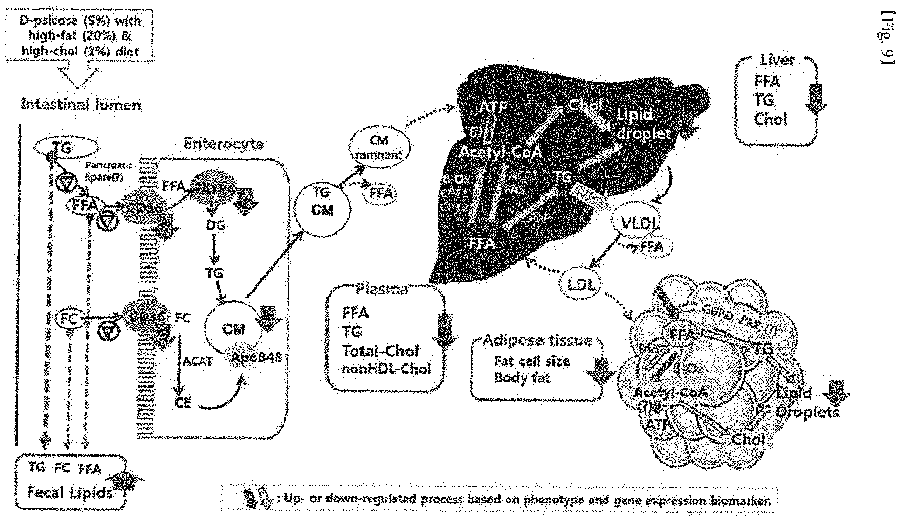

Fig. 9 is a diagram schematically showing the roles of D-psicose on lipid metabolism in the small intestine, liver, and adipose tissues of high-fat diet-induced obese mice, based on the results shown in Figs. 1 to 8.

### [Best Mode]

The present invention provides D-psicose for use in inhibiting the absorption of lipids ingested by a subject and promoting the excretion of the ingested lipids in a subject, thereby preventing hyperlipidemia, arteriosclerosis or fatty liver in the subject, wherein the D-psicose is administered in an amount of 10 to 50 parts by weight, relative to 100 parts by weight of the lipids ingested by the subject.

The present invention also provides a composition comprising D-psicose for use in inhibiting the absorption of lipids ingested by a subject and promoting the excretion of the ingested lipids in a subject, thereby preventing hyperlipidemia, arteriosclerosis or fatty liver in the subject, wherein the D-psicose is administered in an amount of 10 to 50 parts by weight, relative to 100 parts by weight of the lipids ingested by the subject.

The present description, not according to the invention, provides a method for inhibiting lipid absorption and fatty acid synthase (FAC) activity in a subject, comprising administering D-psicose to the subject, an inhibitor of fatty acid synthase comprising D-psicose, a method for preventing or treating hyperlipidemia, arteriosclerosis or fatty liver comprising administering a pharmaceutically effective amount of D-psicose to a subject in need of thereof, and a composition for preventing or treating hyperlipidemia, arteriosclerosis or fatty liver comprising D-psicose.

The composition is intended to include a pharmaceutical composition and a food composition.

Hereinafter, preferred embodiments are presented to assist in understanding the invention.

### Example 1: Influences of D-psicose on body weight, organ weight and adipose tissue weight of high-fat diet-induced obese mice

The following experiment was conducted to investigate the influences of D-psicose on the body weight, organ weight, and adipose tissue weight of high-fat diet-induced obese mice.

First, 4-week-old male C57BL/6J mice (60 total) were purchased from Jackson Laboratory. The animals were acclimated to the vivarium in a thermo-hygrostat (20-23 °C, 45-65%) under a 12 h light/dark cycle and fed a pelletized commercial non-purified diet for 1 week after arrival. The mice were then randomly divided into 6 groups (n=10) and fed the respective experimental diets for 16 weeks: normal diet group (ND, American Institute of Nutrition (AIN)-76 semi-synthetic diet), high-fat diet group (HFD, 20% fat + 1% cholesterol based on AIN-76 diet), PSI group (HFD + 5% D-psicose, w/w, Sigma Chemical Company), ERY group (HFD + 5% erythritol, w/w, Sigma Chemical Company), GLU group (HFD + 5% D-glucose, w/w, Sigma Chemical Company), and FRU group (HFD + 5% D-fructose, w/w, Sigma Chemical Company). All high-fat diet-fed groups were allowed to ingest the same calories by pair feeding based on the PSI group. The mice had *ad libitum* access to distilled water during the experimental period. Their feed intakes and body weights were measured daily and biweekly, respectively. The organ weights and the adipose tissue weights of the mice were measured after sacrificing the animals.

This animal study protocol was approved by the Ethics Committee for Animal Studies at Kyungpook National University, Korea (approval No. KNU 2013-18).

Changes in the body weight of C57BL/6J mice fed both D-psicose and high-fat diet for 16 weeks are shown in Fig. 1 and Table 2. The organ weights and adipose tissue weights of the mice are shown in Table 2.

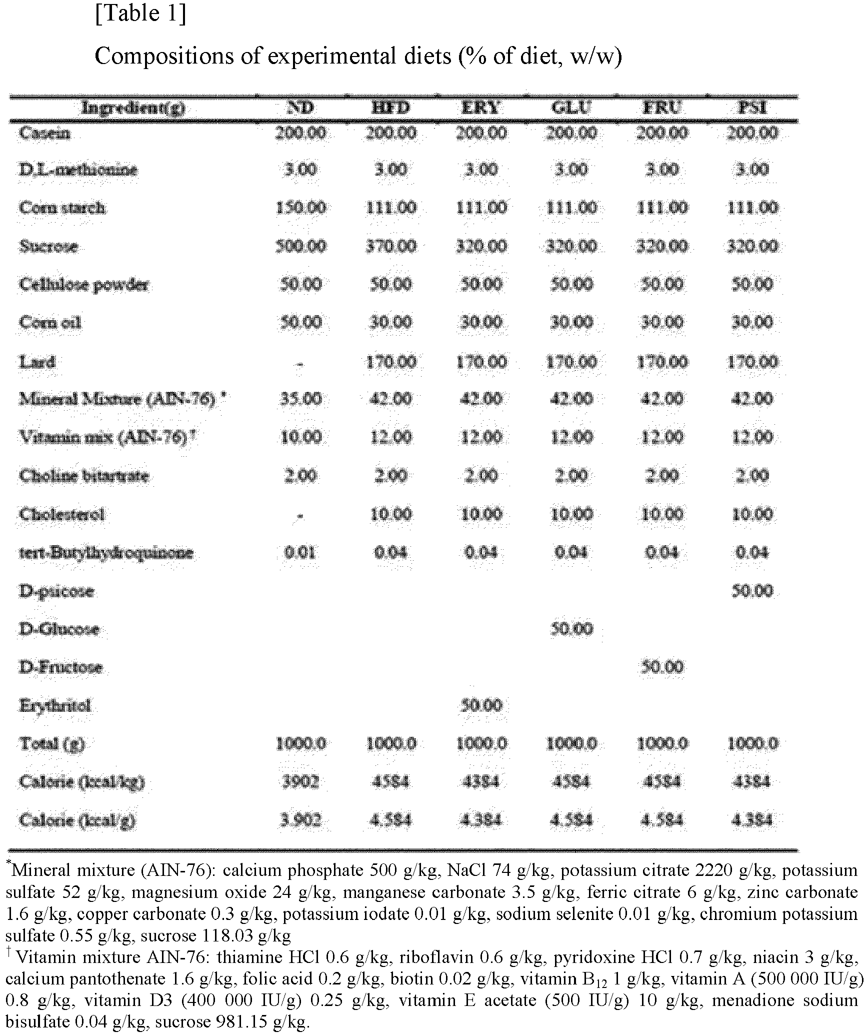

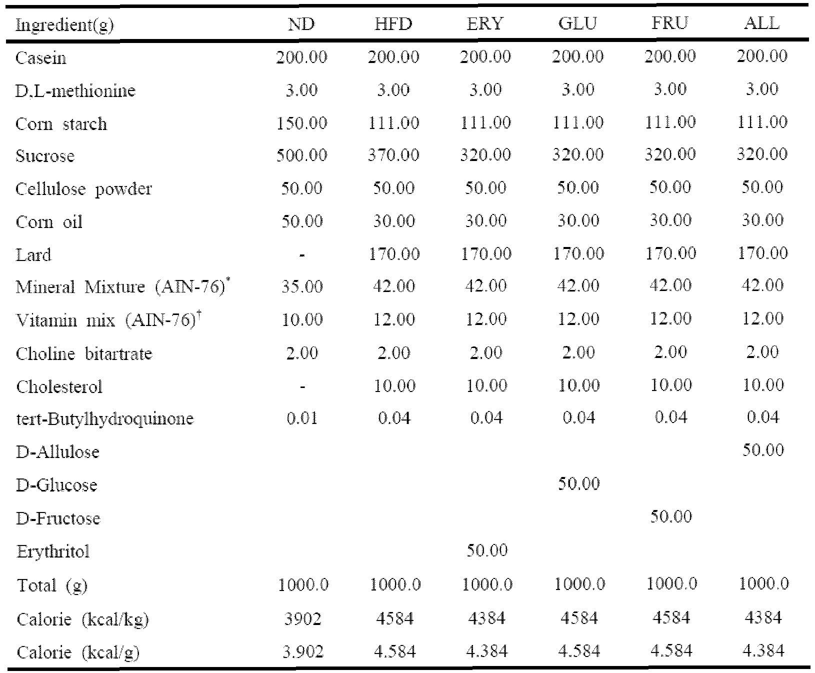

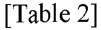

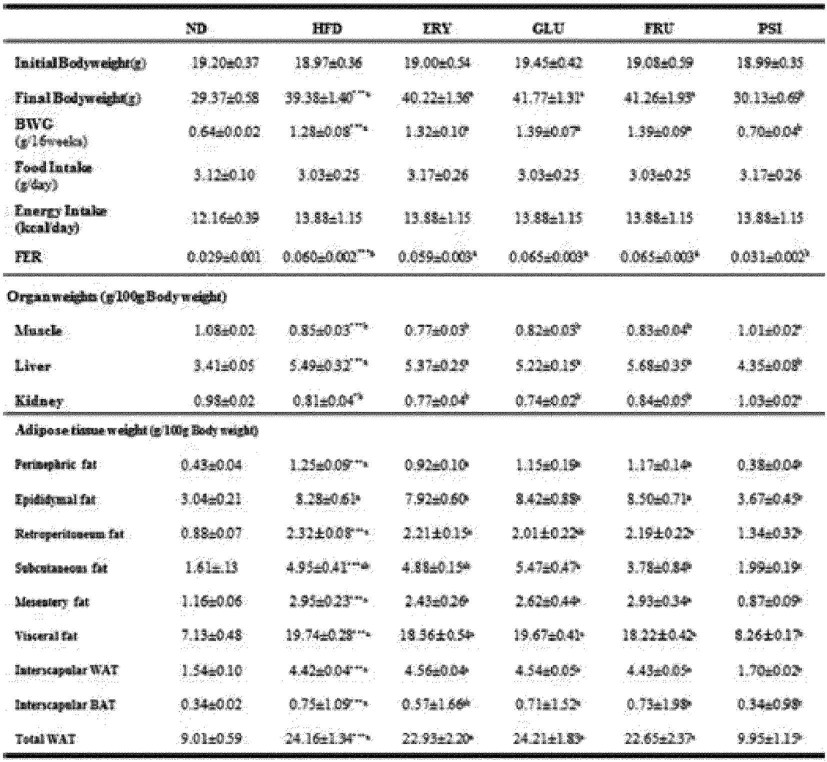

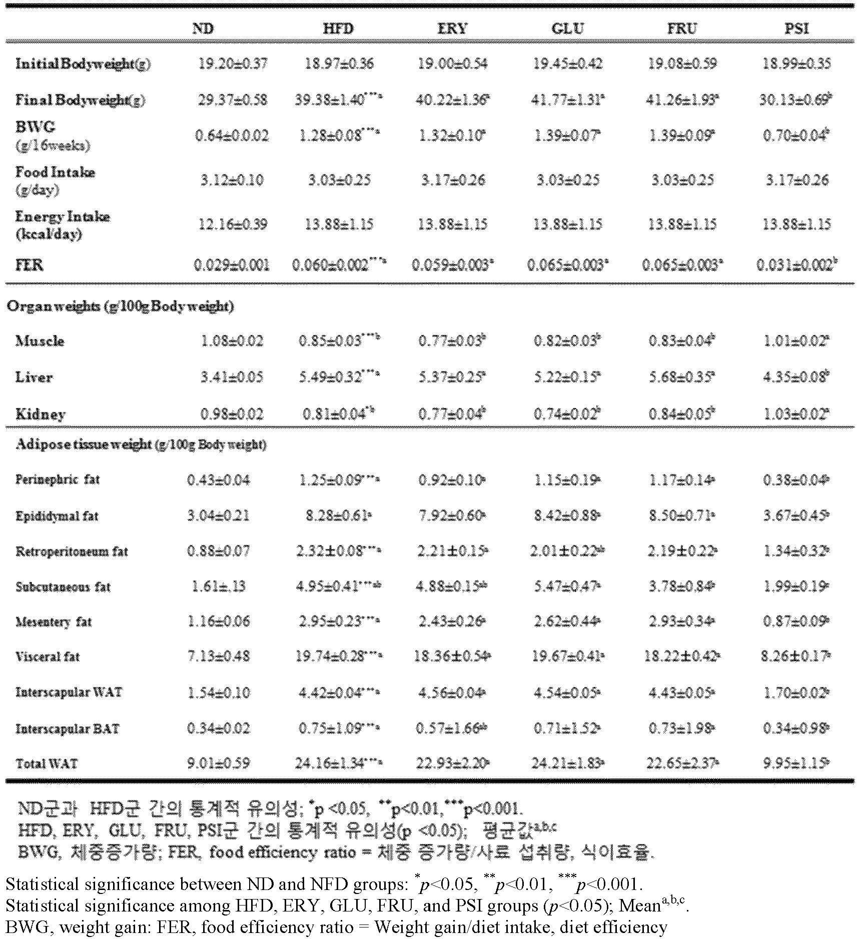

As shown in Fig. 1 and Table 2, the initial body weights of the mice in all experimental groups were almost the same but the body weights of the high-fat diet-induced obese mice increased significantly compared to those of mice in the normal diet group (ND) from 4 weeks after feeding. However, increases in the body weight of the PSI-fed obese mice were considerably inhibited from 4 weeks after diet feeding, and a result, their body weights were maintained at almost the same levels as those of the mice in the ND group. That is, the diet efficiency of the PSI group was significantly lower than those of other high-fat diet groups (HFD, ERY, GLU, and FRU) and was maintained at almost the same level as that of the ND group.

In addition, an investigation was made as to whether the body weight loss of the PSI group was caused by the reduced organ weights. To this end, the weights of the organs (muscles, livers, and kidneys) and adipose tissues (perinephric fat, epididymal fat, retroperitoneal fat, subcutaneous fat, mesenteric fat, visceral fat, interscapular WAT, interscapular BAT, and total WAT) were measured. As shown in Table 2, the weights of the muscles and kidneys per unit body weight of the mouse in the high-fat diet groups (HFD, ERY, GLU, and FRU) except the PSI group decreased significantly compared to those in the ND group and the weights of the livers per unit body weight of the mouse in the high-fat diet groups increased significantly compared to those in the ND group. However, the weights of the muscles and kidneys per unit body weight of the mouse in the PSI group were found to be similar to those in the ND group. The weights of perinephric fat, epididymal fat, retroperitoneal fat, subcutaneous fat, mesenteric fat, visceral fat, interscapular WAT, interscapular BAT, and total WAT per unit body weight of the mouse in the high-fat diet groups (HFD, ERY, GLU, and FRU) except the PSI group increased significantly whereas the weights of all kinds of fats in the PSI group decreased significantly and were maintained at almost the same levels as those in the ND group.

From these results, it can be seen that D-psicose inhibited weight gain in the high-fat diet-induced obese mice and reduced the diet efficiencies and the weights of the livers and adipose tissues per unit body weight of the mouse in the high-fat diet-induced obese mice to levels similar to those of the ND group. In conclusion, D-psicose is effective in normalizing body weight and body fat mass.

### Example 2: Influences of D-psicose on plasma lipid profiles of high-fat diet-induced obese mice

The following experiment was conducted to investigate the influences of D-psicose on the plasma lipid profiles of high-fat diet-induced obese mice.

Plasma free fatty acid, phospholipid, apolipoprotein A-I (Apo A-I), and apolipoprotein B (ApoB B) levels were measured using Nittobo enzymatic kits (Nittobo medical Co., Tokyo, Japan). Plasma HDL-cholesterol, triglyceride (TG), and total cholesterol (total-C) levels were measured using Asan enzymatic kits (Asan, Seoul, South Korea).

The results are shown in Fig. 2 and Table 3.

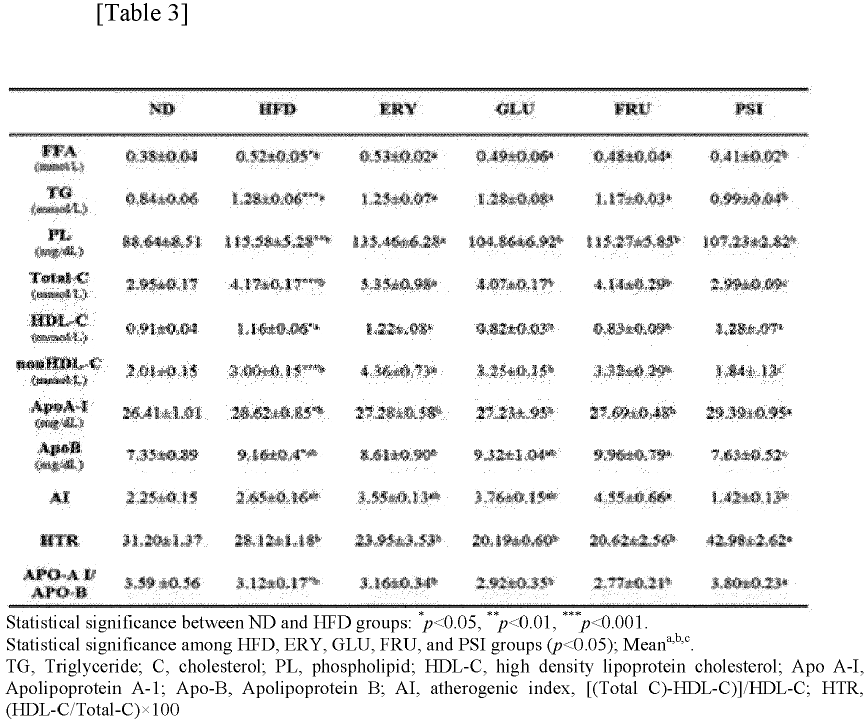

As shown in Fig. 2 and Table 3, the plasma free fatty acid, triglycerides, phospholipid, total-cholesterol, HDL cholesterol, non-HDL cholesterol, apolipoprotein A-I (Apo A-I), and apolipoprotein B (ApoB B) levels in the high-fat diet groups (HFD, ERY, GLU, and FRU) except the PSI group increased significantly compared to those in the ND group but the plasma free fatty acid, triglyceride, total cholesterol, non-HDL cholesterol, and Apo B levels in the PSI group were found to be similar to those in the normal diet group. Particularly, the HDL-cholesterol and Apo A-I levels in the PSI group were higher than those in the ND group and the arteriosclerosis index (AI) of the PSI group was found to be lower than that of the ND group.

From these results, it can be seen that D-psicose reduced the plasma free fatty acid, triglyceride, total cholesterol, non-HDL cholesterol, and Apo B levels in the high-fat diet-induced obese mice to values similar to those in the ND group, thus being effective in normalizing plasma lipid profiles. In addition, D-psicose increased the plasma HDL-cholesterol and Apo A-I levels in the high-fat diet-induced obese mice to higher values than those in the ND group and reduced the arteriosclerosis indices of the high-fat diet-induced obese mice to lower values than those in the ND group. Therefore, it is expected that D-psicose will be used to prevent arteriosclerosis.

### Example 3: Influences of D-psicose on plasma leptin, resistin, and adiponectin levels and leptin:adiponectin ratio (L:A ratio) in high-fat diet-induced obese mice

The following experiment was conducted to investigate the influences of D-psicose on plasma leptin, resistin, and adiponectin levels and leptin:adiponectin ratio (L:A ratio) in high-fat diet-induced obese mice.

Plasma leptin, resistin, and adiponectin levels were measured using Bio-Rad multiplex kits (Hercules, CA, USA). All samples were assayed in duplicate and analyzed using a Luminex 200 labmap system (Luminex, Austin, TX, USA). Data analysis was performed using Bio-Plex Manager software version 4.1.1 (Bio-Rad, Hercules, CA, USA).

The results are shown in Fig. 3.

As shown in Fig. 3, the plasma leptin and resistin levels and the leptin:adiponectin ratios in the high-fat diet groups (HFD, ERY, GLU, and FRU) except the PSI group increased significantly compared to those in the ND group but the plasma leptin and resistin levels and the leptin:adiponectin ratio in the PSI group were reduced considerably to levels similar to those in the ND group.

From these results, it can be seen that D-psicose reduced the plasma leptin and resistin levels and the leptin:adiponectin ratios in the high-fat diet-induced obese mice to normal values.

### Example 4: Influences of D-psicose on hepatic lipid profiles, hepatic lipid regulating enzyme activities, and hepatic tissue morphologies of high-fat diet-induced obese mice

The following experiment was conducted to investigate the influences of D-psicose on hepatic lipid profiles, hepatic lipid regulating enzyme activities, and hepatic tissue morphologies of high-fat diet-induced obese mice.

### Example 4-1. Hepatic lipid profiles

Hepatic lipids were extracted from mice in the normal diet group (ND) and high-fat diet groups (HFD, ERY, GLU, FRU, and PSI) and dried. Then, each of the dried hepatic lipid extracts was dissolved in 1 ml of ethanol. 200 µl of the lipid solution was emulsified in a solution of Triton X-100 and sodium cholate in distilled water. Hepatic fatty acid, triglyceride, and cholesterol levels were analyzed using the same enzymatic kits as those used in Example 2.

The results are shown in (A) of Fig. 4.

As shown in (A) of Fig. 4, the hepatic fatty acid, triglyceride, and cholesterol levels in the high-fat diet groups (HFD, ERY, GLU, FRU, and PSI) were found to be significantly higher than those in the ND group but the hepatic fatty acid, triglyceride, and cholesterol levels in the PSI group decreased significantly compared to those in other high-fat diet groups (HFD, ERY, GLU, and FRU).

### Example 4-2. Hepatic lipid regulating enzyme activity

Samples were prepared and analyzed according to the method developed by Hulcher and Oleson. Specifically, the activity of fatty acid synthase (FAS) as a hepatic lipid regulating enzyme was measured by spectrophotometric assay according to the method described by Nepokroeff et al. Each sample was mixed with 100 µl of cytoplasmic fraction and the mixture was allowed to react at 30 °C for 2 min. A reduction in absorbance at 340 nm was measured. Fatty acid synthase (FAS) activity units were expressed as nanomoles (nmol) of NADPH oxidized for 1 min per mg of cytoplasmic fraction. Fatty acid β-oxidation activity was measured by monitoring the reduction of NAD⁺ to NADH in the presence of palmitoyl-CoA, as described by Lazarow. β-oxidation activity units were expressed as nanomoles (nmol) of NADH produced for 1 min per mg of mitochondrial protein.

The results are shown in (B) of Fig. 4.

As shown in (B) of Fig. 4, the FAS activities and fatty acid β-oxidation activities in the high-fat diet groups (HFD, ERY, GLU, and FRU) except the PSI group increased significantly compared to those in the ND group but the FAS activities and fatty acid β-oxidation activities in the PSI group decreased significantly compared to those in other high-fat diet groups and were found to be similar to those in the ND group.

### Example 4-3. Hepatic tissue morphologies

Liver tissues were removed from the mice in the normal diet group (ND) and high-fat diet groups (HFD, ERY, GLU, FRU, and PSI) and fixed in a buffer solution of 10% formalin. The fixed liver tissues were embedded in paraffin. 4-mm sections were prepared from the liver tissues and their cross-sections were dyed with hematoxylin and eosin. Stained areas were observed using an optical microscope at a magnification of 200× (Nikon, Tokyo, Japan).

The results are shown in (C) of Fig. 4.

As shown in (C) of Fig. 4, the accumulation of adipocytes in the liver tissues of the high-fat diet groups (HFD, ERY, GLU, and FRU) except the PSI group was more distinctly observed than in the liver tissues of the ND group and the size of adipocytes in the liver tissues of the PSI group was smaller than that in the liver tissues of other high-fat diet groups.

These results reveal that D-psicose decreased the levels of fatty acids, triglycerides, and cholesterol, FAS activities, and adipocyte sizes in the livers of the high-fat diet-induced obese mice. In conclusion, D-psicose is effective in inhibiting fatty liver. In addition, D-psicose reduced hepatic fatty acid β-oxidation activities, which had been increased by high-fat diets, to levels similar to those in the normal diet group. In conclusion, D-psicose is effective in maintaining the homeostasis of hepatic lipid metabolism at the normal level.

### Example 5: Influences of D-psicose on lipid regulating enzyme activities and tissue morphologies in adipose tissues of high-fat diet-induced obese mice

The following experiment was conducted to investigate the influences of D-psicose on lipid regulating enzyme activities and tissue morphologies in the adipose tissues of high-fat diet-induced obese mice.

### Example 5-1. Lipid regulating enzyme activities in adipose tissues

Samples were prepared and analyzed according to the method developed by Hulcher and Oleson. Specifically, the activity of fatty acid synthase (FAS) as a lipid regulating enzyme of epididymal white adipose tissue was measured by spectrophotometric assay according to the method described by Nepokroeff et al. Each sample was mixed with 100 µl of cytoplasmic fraction and the mixture was allowed to react at 30 °C for 2 min. A reduction in absorbance at 340 nm was measured. FAS activity units were expressed as nanomoles (nmol) of NADPH oxidized for 1 min per mg of cytoplasmic fraction. Fatty acid β-oxidation activity was measured by monitoring the reduction of NAD⁺ to NADH in the presence of palmitoyl-CoA, as described by Lazarow. β-oxidation activity units were expressed as nanomoles (nmol) of NADH produced for 1 min per mg of mitochondrial protein.

The results are shown in (A) of Fig. 5.

As shown in (A) of Fig. 5, the FAS activities in the high-fat diet groups (HFD, ERY, GLU, and FRU) except the PSI group increased significantly compared to those in the ND group and the fatty acid β-oxidation activities in the high-fat diet groups (HFD, ERY, GLU, and FRU) except the PSI group decreased significantly compared to those in the ND group. The FAS activities in the PSI group decreased significantly compared to those in other high-fat diet groups whereas the fatty acid β-oxidation activities in the PSI group increased significantly compared to those in other high-fat diet groups and were found to be similar to those in the normal diet group. From these results, it can be seen that D-psicose reduced the synthesis of fatty acids and increased the oxidation of fatty acids in the adipose tissues of high-fat diet-induced obese mice. In conclusion, D-psicose is effective in reducing body fat mass.

### Example 5-2. Adipose tissue morphologies

Epididymal WATs were removed from the mice in the normal diet group (ND) and high-fat diet groups (HFD, ERY, GLU, FRU, and PSI) and fixed in a buffer solution of 10% formalin. The fixed epididymal WATs were embedded in paraffin. 4-mm sections were prepared from the epididymal WATs and their cross-sections were dyed with hematoxylin and eosin. Stained areas were observed using an optical microscope at a magnification of 200× (Nikon, Tokyo, Japan).

The results are shown in (B) of Fig. 5.

As shown in (B) of Fig. 5, an increase in the size of adipocytes in the epididymal WATs of the high-fat diet groups (HFD, ERY, GLU, and FRU) except the PSI group was distinctly observed compared to in the epididymal WATs of the ND group. The size of adipocytes in the PSI group was found to be relatively small compared to that in the other high-fat diet groups.

From these results, it can be seen that D-psicose reduced the synthesis of fatty acids and increased the oxidation of fatty acids in the adipose tissues of high-fat diet-induced obese mice, resulting in a reduction in the size of adipocytes and an inhibition of lipid accumulation. In conclusion, D-psicose is effective in normalizing body fat mass to the normal level.

### Example 6: Influences of D-psicose on mRNA expression of genes involved in fatty acid synthesis and oxidation in the livers of high-fat diet-induced obese mice

The following experiment was conducted to investigate the influences of D-psicose on mRNA expression of genes (FAS, ACC1, CPT1α, and CPT2) involved in fatty acid synthesis and oxidation in the livers of high-fat diet-induced obese mice.

Samples were prepared and analyzed as previously described. Specifically, total RNA was synthesized into cDNA using a QuantiTect Reverse Transcription kit (QIAGEN Gmblh, Hilden, Germany). RNA expression was quantified with real-time quantitative PCR using a QuantiTect SYBR Green PCR kit (QIAGEN Gmblh, Hilden, Germany). Primers were designed to detect FAS (fatty acid synthase, 14101), ACC1 (Acetyl-CoA carboxylase 1, 107476), CPT1α (Carnitine palmitoyltransferase 1α, 12894), and CPT2 (Carnitine palmitoyltransferase 2, 12896). GAPDH was used as an internal transcription marker. The reaction was performed a total of 40 cycles (each consisting of 15 sec at 94 °C, 30 sec at 58 °C, 30 sec at 72 °C, and 15 sec at 65 °C). Fluorescence signals were monitored every cycle and the resulting threshold cycles (Ct) were analyzed. mRNA expression in each experimental group was quantified using a CFX96 Real time system (Bio-rad, USA).

The results are shown in Fig. 6.

As shown in Fig. 6, mRNA expression levels of genes (FAS and ACC1) involved in hepatic fatty acid synthesis and genes (CPT1α and CPT2) involved in fatty acid oxidation in all high-fat diet groups (HFD, ERY, GLU, FRU, PSI) were significantly lower than those in the ND group. Particularly, mRNA expression levels of genes (FAS and ACC1) involved in hepatic fatty acid synthesis and genes (CPT1α and CPT2) involved in fatty acid oxidation in the PSI group were much significantly lower than those in the ND group.

From these results, it can be seen that D-psicose reduced mRNA expression of genes involved in fatty acid synthesis in the livers of high-fat diet-induced obese mice to inhibit fat production in the livers.

### Example 7: Influences of D-psicose on lipid excretion in feces from high-fat diet-induced obese mice

The following experiment was conducted to investigate the influences of D-psicose on lipid excretion in feces from high-fat diet-induced obese mice.

Lipids were extracted from feces from mice in the normal diet group (ND) and the high-fat diet groups (HFD, ERY, GLU, FRU, and PSI) and dried. Then, each of the dried lipid extracts was dissolved in 1 ml of ethanol. 200 µl of the lipid solution was emulsified in a solution of Triton X-100 and sodium cholate in distilled water. Triglyceride, cholesterol, and fatty acid levels in the feces were analyzed using the same enzymatic kits as those used in Example 2.

The results are shown in Fig. 7.

As shown in Fig. 7, the triglyceride, cholesterol, and fatty acid levels in the feces from the high-fat diet groups (HFD, ERY, GLU, FRU, and PSI) were found to be significantly higher than those from the normal diet group. Particularly, the triglyceride, cholesterol, and fatty acid levels in the feces from the PSI group were confirmed to be significantly higher than those from other high-fat diet groups (HFD, ERY, GLU, and FRU).

From these results, it can be seen that D-psicose increased lipid excretion in feces from high-fat diet-induced obese mice, which is associated with the inhibitory effect of D-psicose on enteric fat absorption.

### Example 8: Influences of D-psicose on mRNA expression of genes involved in lipid absorption in the small intestines of high-fat diet-induced obese mice

The following experiment was conducted to investigate the influences of D-psicose on mRNA expression of genes (CD36, FATP4, and ApoB48) involved in lipid absorption and genes (ABCG5 and ABCG8) involved in excretion in the small intestines of high-fat diet-induced obese mice.

Samples were prepared and analyzed as previously described. Specifically, total RNA was synthesized into cDNA using a QuantiTect Reverse Transcription kit (QIAGEN Gmblh, Hilden, Germany). RNA expression was quantified with real-time quantitative PCR using a QuantiTect SYBR Green PCR kit (QIAGEN Gmblh, Hilden, Germany). Primers were designed to detect CD36 (cluster of differentiation 36, 12491), ApoB48 (apolipoprotein B 48, 238055), FATP4 (fatty acid transporter 4, 26569), ABCG5 (ATP-binding cassette sub-family G member5, 27409), and ABCG8 (ATP-binding cassette sub-family G member8, 67470). GAPDH was used as an internal transcription marker. The reaction was performed a total of 40 cycles (each consisting of 15 sec at 94 °C, 30 sec at 58 °C, 30 sec at 72 °C, and 15 sec at 65 °C). Fluorescence signals were monitored every cycle and the resulting threshold cycles (Ct) were analyzed. mRNA expression in each experimental group was quantified using a CFX96 Real time system (Bio-rad, USA).

The results are shown in Fig. 8.

As shown in Fig. 8, mRNA expression levels of genes (CD36, FATP4, and ApoB48) involved in lipid absorption in the small intestine were significantly higher in the high-fat diet groups (HFD, ERY, GLU, and FRU) except the PSI group than those in the ND group. mRNA expression levels of genes (CD36, FATP4, and Apo B48) involved in lipid absorption in the small intestine were significantly lower in the PSI group than those in other high-fat diet groups (HFD, ERY, GLU, and FRU) and were maintained at the same levels as those in the normal diet group.

From these results, it can be seen that D-psicose reduced mRNA expression of genes involved in lipid absorption in the small intestines of high-fat diet-induced obese mice. In conclusion, D-psicose has an inhibitory effect on lipid availability because of its ability to inhibit lipid absorption in the small intestine.

Based on the results obtained in Examples 1-8, the roles of D-psicose on lipid metabolism in the small intestines, hepatic tissues, and adipose tissues of high-fat diet-induced obese mice are briefly summarized in Fig. 9.

The composition was prepared into the following formulations.

### Preparation Example 1: Preparation of pharmaceutical formulations

### 1. Preparation of powders

| | |
|---|---|
| D-psicose | 200 mg |
| Lactose | 100 mg |

The ingredients were mixed together and filled in air-tight bags to prepare powders

### 2. Preparation of tablets

| | |
|---|---|
| D-psicose | 200 mg |
| Corn starch | 100 mg |
| Lactose | 100 mg |
| Magnesium stearate | 2 mg |

The ingredients were mixed together and compressed to prepare tablets according to a suitable method known in the art.

### 3. Preparation of capsules

| | |
|---|---|
| D-psicose | 200 mg |
| Corn starch | 100 mg |
| Lactose | 100 mg |
| Magnesium stearate | 2 mg |

The ingredients were mixed together and filled in gelatin capsules to prepare capsules according to a suitable method known in the art.

### 4. Preparation of injectables

| | |
|---|---|
| D-psicose | 200 mg |
| Mannitol | 100 mg |
| Na₂HPO₄·12H₂O | 2 mg |
| Sterilized distilled water for injection | q.s |

The ingredients were mixed together in ampules (2 ml each) to prepare injectables according to a suitable method known in the art.

### Preparation Example 2 : Preparation of foods

Foods including D-psicose were prepared by the following procedures:
1. Preparation of cooking sauces
   Healthy cooking sauces including 20-95 wt% of D-psicose were prepared.
2. Preparation of tomato ketchups and sauces
   Healthy tomato ketchups and sources including 0.2-1.0 wt% of D-psicose were prepared.
3. Preparation of flour foods
   0.5-5.0 wt% of D-psicose was added to flour. The mixture was used to prepare breads, cakes, cookies, crackers, and healthy flour products.
4. Preparations of soups and gravies
   0.1-5.0 wt% of D-psicose was added to soups and gravies for healthy meat processed products and flour products.
5. Preparation of ground beef
   Healthy ground beef including 10 wt% of D-psicose was prepared.
6. Preparation of dairy products
   5-10 wt% of D-psicose was added to milk. The mixture was used to prepare dairy products such as butters and ice creams.

### Preparation Example 3 : Preparation of beverages

1. Preparation of carbonated beverages
   10-15% of D-psicose, 5-10% of sugar, 0.05-0.3% of citric acid, 0.005-0.02% of caramel, and 0.1-1% of vitamin were mixed together. The mixture was mixed with 75-80% of purified water to prepare a syrup. The syrup was sterilized at 85-98 °C for 20-180 sec and mixed with cooling water in a ratio of 1:4. The mixture was injected with 0.5-0.82% of carbonic acid gas to prepare a carbonated beverage containing D-psicose.
2. Preparation of healthy beverages
   D-psicose (solid content: 2.5%, 97.16%), jujube extract (65 brix, 2.67%), fruit-beverage complex extract (solid content: 70%, 0.12%), vitamin C (0.02%), calcium pantothenate (0.02%), and licorice extract (solid content: 65%, 0.01%) were mixed together. The mixture was homogenized, instantaneously sterilized, and packaged in small packaging containers such as glass bottles and PET bottles to prepare healthy beverages.
3. Preparation of vegetable juices
   0.5 g of D-psicose was added to 1,000 ml of a tomato or carrot juice to prepare a healthy vegetable juice.
4. Preparation of fruit juices
   0.1 g of D-psicose was added to an apple or grape juice to prepare a healthy fruit juice.

### [Industrial Applicability]

In the present invention, the physiological activity of D-psicose was clarified by assigning isocaloric diets to diet groups to exclude the effect of D-psicose on calorie reduction. As a result, it was found that D-psicose has functions of inhibiting lipid absorption in the small intestine and considerably increasing lipid levels in feces to inhibit fat production and reduces body weight, body fat mass, and plasma lipid levels such that body weight, body fat mass, and plasma lipid profiles are normalized in a short time. Due to these advantages, it is expected that D-psicose will be used to prevent and/or treat lipid-related metabolic diseases.

## Claims

1. D-psicose for use in inhibiting the absorption of lipids ingested by a subject and promoting the excretion of the ingested lipids in a subject, thereby preventing hyperlipidemia, arteriosclerosis or fatty liver in the subject, wherein the D-psicose is administered in an amount of 10 to 50 parts by weight, relative to 100 parts by weight of the lipids ingested by the subject.

2. Composition comprising D-psicose for use in inhibiting the absorption of lipids ingested by a subject and promoting the excretion of the ingested lipids in a subject, thereby preventing hyperlipidemia, arteriosclerosis or fatty liver in the subject, wherein the D-psicose is administered in an amount of 10 to 50 parts by weight, relative to 100 parts by weight of the lipids ingested by the subject.

## Patentansprüche

1. D-Picose zur Verwendung beim Hemmen der Absorption von Lipiden, die von einem Subjekt aufgenommen werden, und beim Fördern der Ausscheidung der aufgenommenen Lipide bei einem Subjekt, wodurch Hyperlipidämie, Arteriosklerose oder Fettleber bei dem Subjekt verhindert werden, wobei die D-Picose in einer Menge von 10 bis 50 Gewichtsteilen, bezogen auf 100 Gewichtsteile der Lipide, die von dem Subjekt aufgenommen werden, verabreicht wird.

2. Zusammensetzung, umfassend D-Picose zur Verwendung beim Hemmen der Absorption von Lipiden, die von einem Subjekt aufgenommen werden, und beim Fördern der Ausscheidung der aufgenommenen Lipide bei einem Subjekt, wodurch Hyperlipidämie, Arteriosklerose oder Fettleber bei dem Subjekt verhindert werden, wobei die D-Picose in einer Menge von 10 bis 50 Gewichtsteilen, bezogen auf 100 Gewichtsteile der Lipide, die von dem Subjekt aufgenommen werden, verabreicht wird.

## Revendications

1. D-psicose pour utilisation dans l'inhibition de l'absorption des lipides ingérés par un sujet et favoriser l'excrétion des lipides ingérés chez un sujet, prévenant ainsi l'hyperlipidémie, l'artériosclérose ou la stéatose hépatique chez le sujet, **caractérisé en ce que** le D-psicose est administré en une quantité de 10 à 50 parties en poids, par rapport à 100 parties en poids des lipides ingérés par le sujet.

2. Composition comprenant du D-psicose pour utilisation dans l'inhibition de l'absorption des lipides ingérés par un sujet et favoriser l'excrétion des lipides ingérés chez un sujet, prévenant ainsi l'hyperlipidémie, l'artériosclérose ou la stéatose hépatique chez le sujet, **caractérisé en ce que** le D-psicose est administré à raison de 10 à 50 parties en poids, par rapport à 100 parties en poids des lipides ingérés par le sujet.
